# EUROPEAN PATENT SPECIFICATION

(11) **EP 4 475 944 B1**
(45) Date of publication and mention of the grant of the patent: **11.11.2026**
(21) Application number: 23709008.9
(22) Date of filing: 07.02.2023
(51) Int. Cl.: A61N 1/378, A61N 1/372

(54) **TISSUE STIMULATION SYSTEMS SUCH AS FOR PACING CARDIAC TISSUE**
GEWEBESTIMULATIONSSYSTEME WIE ZUM BEISPIEL ZUR STIMULATION VON HERZGEWEBE
SYSTÈMES DE STIMULATION TISSULAIRE PAR EXEMPLE POUR LA STIMULATION DE TISSU CARDIAQUE

(30) Priority: 10.02.2022 US 202217668771
(43) Date of publication of application: 18.12.2024
(73) Proprietor: EBR Systems, Inc., Sunnyvale, CA 94085 (US)
(72) Inventor: WILLIS, Parker, Sunnyvale, California 94085 (US); RILEY, Richard, Sunnyvale, California 94085 (US); FAYRAM, Timothy A., Sunnyvale, California 94085 (US)
(74) Representative: Mathys & Squire
(86) International application number: PCT/US2023/012534
(87) International publication number: WO 2023/154288

(56) References cited:
- US-A1- 2010 228 308
- US-A1- 2012 203 306
- US-A1- 2019 313 908
- US-A1- 2020 238 093

## Description

### TECHNICAL FIELD

The present technology generally relates to implantable medical devices and, in particular embodiments, to leadless tissue stimulation systems and methods for pacing cardiac tissue.

### BACKGROUND

Electrical stimulation of body tissue is used throughout medicine for treatment of both chronic and acute conditions. Among many examples, peripheral muscle stimulation is reported to accelerate healing of strains and tears, bone stimulation is likewise indicated to increase the rate of bone regrowth/repair in fractures, and nerve stimulation is used to alleviate chronic pain. Further there is encouraging research in the use of electrical stimulation to treat a variety of nerve and brain conditions, such as essential tremor, Parkinson's disease, migraine headaches, functional deficits due to stroke, and epileptic seizures.

Cardiac pacemakers and implantable defibrillators are examples of commonly implanted device utilizing electrical stimulation to stimulate cardiac and other tissues. A pacemaker is a battery-powered electronic device implanted under the skin, connected to the heart by an insulated metal lead wire with a tip electrode. Pacemakers were initially developed for and are most commonly used to treat slow heart rates (bradycardia), which may result from a number of conditions. More recently, advancements in pacemaker complexity, and associated sensing and pacing algorithms have allowed progress in using pacemakers for the treatment of other conditions, notably heart failure (HF) and fast heart rhythms (tachyarrhythmia/tachycardia).

Electrical energy sources connected to electrode/lead wire systems have typically been used to stimulate tissue within the body. The use of lead wires is associated with significant problems such as complications due to infection, lead failure, and electrode/lead dislodgement. The requirement for leads in order to accomplish stimulation also limits the number of accessible locations in the body. The requirement for leads has also limited the ability to stimulate at multiple sites (multisite stimulation).

In US 2012/203306, a time-reversal acoustics system is disclosed which includes a transmitter configured to send a high intensity acoustic waveform signal focused on an implantable receiver. The receiver includes a piezoelectric transducer configured to convert received acoustic energy to an electrical signal used to energize an internal electrical circuit. Such circuit may be used to operate at least one tissue stimulating electrode, at least one sensor such as an ECG sensor, charge an internal battery or perform another useful function. The system may be used as a wireless cardiac pacemaker or a neurostimulator.

US 2010/228308 describes systems including an implantable receiver-stimulator and an implantable controller-transmitter used for leadless electrical stimulation of body tissues. Cardiac pacing and arrhythmia control is accomplished with one or more implantable receiver-stimulators and an external or implantable controller-transmitter. Systems are implanted by testing external or implantable devices at different tissue sites, observing physiologic and device responses, and selecting sites with preferred performance for implanting the systems. In these systems, a controller-transmitter is activated at a remote tissue location to transmit/deliver acoustic energy through the body to a receiver-stimulator at a target tissue location. The receiver-stimulator converts the acoustic energy to electrical energy for electrical stimulation of the body tissue. The tissue locations(s) can be optimized by moving either or both of the controller-transmitter and the receiver-stimulator to determine the best patient and device responses.

US 2020/238093 describes devices, systems, and methods for providing electrical stimulation to the left ventricle of a human heart in a patient suffering from Left Bundle Branch Block (LBBB). In particular, including an implantable receiver-stimulator and an implantable controller-transmitter for leadless electrical stimulation of the heart. The receiver-stimulator can include one or more sensors capable of detecting the electrical conduction of the heart and the receiver-stimulator can be configured to pace the stimulation of the left ventricle based off the sensed electrical conduction to achieve synchronization of the left and right ventricles.

US 2019/313908 describes a reconfigurable implantable system for ultrasonic power control and telemetry includes a charging device that includes an ultrasonic transducer to transmit and receive ultrasonic signals transmitted through a biological body, and a signal generator to drive the ultrasonic transducer to transmit an ultrasonic charging signal through the biological body. The system further includes an implantable device configured to communicate wirelessly with the charging device through the biological body via an ultrasonic communication link between the implantable device and the charging device. An implantable ultrasonic transducer receives the ultrasonic charging signal from the charging device and transmits ultrasonic signals through the biological body. A power unit coupled to the ultrasonic transducer harvests energy from the received ultrasonic charging signal when the implantable device is in an energy harvesting mode. A communication unit is configured to switch the implantable device between the energy harvesting mode and an ultrasonic communication mode, and to read data from the sensing or actuation unit and transmit the data through the implantable ultrasonic transducer when the implantable device is in the ultrasonic communication mode.

### Summary of the invention

The invention is defined in the appended claims. Aspects, embodiments and examples disclosed herein which do not fall within the scope of the appended claims do not form part of the invention, and are merely provided for illustrative purposes.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure.
Figure 1 is a schematic diagram of a tissue stimulation system configured in accordance with embodiments of the present technology.
Figure 2 is a schematic diagram of a tissue stimulation system configured in accordance with additional embodiments of the present technology.
Figure 3 illustrates an energy-harvesting circuit of a receiver-stimulator of the system of Figure 2 configured in accordance with embodiments of the present technology.
Figure 4 is a schematic diagram of a tissue stimulation system configured in accordance with additional embodiments of the present technology.
Figure 5 is a is a schematic diagram of a tissue stimulation system configured in accordance with additional embodiments of the present technology
Figure 6 is a flow diagram of a process or method for a tissue stimulation system to generate and deliver electrical stimulation (e.g., electrical pacing pulses) to the heart of a patient in accordance with embodiments of the present technology.
Figure 7 is a partially schematic diagram of a tissue stimulation system configured in accordance with additional embodiments of the present technology.
Figure 8 illustrates an example control scheme and timing sequence for the system of Figure 7 in a pacemaker application in accordance with embodiments of the present technology.

### DETAILED DESCRIPTION

Aspects of the present disclosure are directed generally to systems and methods for stimulating tissue of a patient, such as heart tissue for cardiac pacing. In several of the embodiments described below, for example, a system for stimulating (e.g., pacing) the heart of a patient includes a controller-transmitter and one or more receiver-stimulators in operable communication with one another. The receiver-stimulator can be implanted at the heart of the patient and the controller-transmitter can be implanted in the patient remote from the heart. The controller-transmitter can be configured to transmit acoustic signals to the receiver-stimulator including acoustic energy and modulated and/or encoded stimulation parameters. The receiver-stimulator can receive the acoustic signals and convert the acoustic signals to electrical energy for delivery to the heart via one or more stimulation electrodes according to the stimulation parameters. The receiver-stimulator can further be configured to transmit a radiofrequency signal to the controller-transmitter including information about sensed physiological parameters of the patient, status information, and the like. In some embodiments, the controller-transmitter can receive the radiofrequency signal and modify the transmitted acoustic signals accordingly. In some embodiments, the controller-transmitter can further transmit a radiofrequency signal to the receiver-stimulator, and the receiver-stimulator can harvest energy from the radiofrequency signal to power one or more electronic components thereof (e.g., a controller, a physiological sensor).

In several additional embodiments described below, a method of delivering electrical stimulation energy to the heart of a patient can include receiving a cardiac signal indicative of a function of the heart from, for example, the receiver-stimulator or the controller-transmitter. The method can further include determining a temporal spacing between a selected number of beats of the heart based on the received cardiac signal, and determining an average temporal spacing between the beats to determine a pacing pulse interval. The method can then include delivering the electrical stimulation to the heart according to the pacing pulse interval. In some aspects of the present technology, determining the temporal spacing does not include (e.g., is not dependent on) determining an amplitude or morphology of a waveform of the cardiac signal.

In several additional embodiments described below, an implanted receiver-stimulator includes a storage unit configured to store electrical energy converted from acoustic signals transmitted by a controller-transmitter. The storage unit can be electrically coupled to one or more stimulation electrodes via a switch. The receiver-stimulator can further include a controller electrically coupled to the switch and configured to control the switch to close and open one or more times to deliver the electrical energy from the storage unit to the stimulation electrodes for output to the heart as a first electrical pulse and a second electrical pulse. The controller-transmitter can be configured to detect the first electrical pulse and to cease transmission of the acoustic signal in response to detecting the first electrical pulse. In some embodiments, the controller can control the switch to deliver the first stimulation pulse based on a charge state of the storage unit such that, for example, the controller-transmitter ceases transmission of the acoustic signal when the storage unit is sufficiently charged. The second electrical pulse can have an amplitude, pulse duration, and/or other characteristic sufficient to stimulate the heart of the patient.

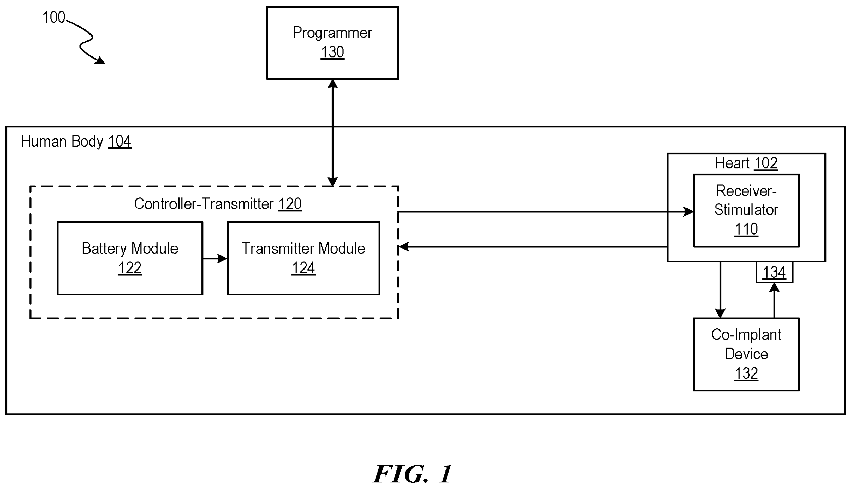

Specific details of several embodiments of the present technology are described herein with reference to Figures 1-8. The present technology, however, can be practiced without some of these specific details. In some instances, well-known structures and techniques often associated with leadless tissue stimulation systems, cardiac pacing, electronic circuitry, acoustic and radiofrequency transmission and receipt, and the like, have not been shown in detail so as not to obscure the present technology. The terminology used in the description presented below is intended to be interpreted in its broadest reasonable manner, even though it is being used in conjunction with a detailed description of certain specific embodiments of the disclosure. Certain terms can even be emphasized below; however, any terminology intended to be interpreted in any restricted manner will be overtly and specifically defined as such in this Detailed Description section.

The accompanying Figures depict embodiments of the present technology and are not intended to be limiting of its scope. The sizes of various depicted elements are not necessarily drawn to scale, and these various elements can be arbitrarily enlarged to improve legibility. Component details can be abstracted in the Figures to exclude details such as position of components and certain precise connections between such components when such details are unnecessary for a complete understanding of how to make and use the present technology. Many of the details, dimensions, angles, and other features shown in the Figures are merely illustrative of particular embodiments of the disclosure. Accordingly, other embodiments can have other details, dimensions, angles, and features without departing from the spirit or scope of the present technology.

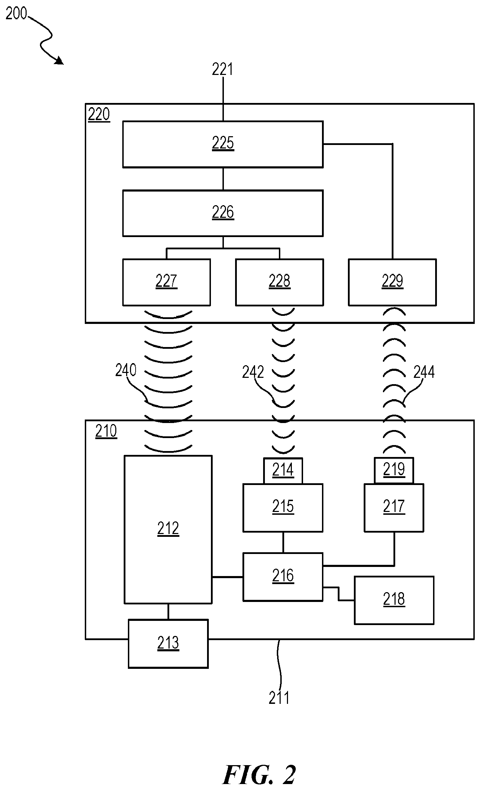

General aspects of the systems and methods of the present technology, and the environments in which the present technology can operate, are described below with reference to Figure 1. Further embodiments of the technology are described below with reference to Figures 2-8. One of ordinary skill in the art will appreciate that the components and functionalities of the various embodiments can be combined with one another. For example, any of the receiver-stimulators and/or controller-transmitters and associated methods described in detail with reference to Figures 2-8 can be combined with one another and/or operate in the environments described with reference to Figure 1. Moreover, while the present technology is generally described in the environment of stimulating the heart, one of ordinary skill in the art will understand that one or more aspects of the present technology are applicable to other implantable devices configured to treat other areas of the human body.

Figure 1 is a schematic diagram of a tissue stimulation system 100 ("system 100") configured in accordance with embodiments of the present technology. In the illustrated embodiment, the system 100 is configured to stimulate a heart 102 within a body 104 of a human patient. The system 100 can include a receiver-stimulator 110 (which can also be referred to as a stimulator, ultrasound receiver, an acoustic receiver, and the like) in operable communication (e.g., wireless and/or radio communication) with a controller-transmitter 120 (which can also be referred to as an ultrasound transmitter, a pulse generator, an acoustic transmitter, and the like). The controller-transmitter 120 can include a battery module 122 and a transmitter module 124 operably coupled to and powered via the battery module 122. In some embodiments, both the receiver-stimulator 110 and the controller-transmitter 120 can be implanted within the body 104 of the human patient. For example, the receiver-stimulator 110 can be implanted at and/or proximate the heart 102 (e.g., in the left ventricle, the right ventricle, or proximate area) for delivering stimulation pulses to the heart 102, while the controller-transmitter 120 can be positioned at another location remote from the heart 102 (e.g., in the chest area). As described in greater detail below with reference to Figures 2-8, the transmitter module 124 of the controller-transmitter 120 can direct energy (e.g., acoustic energy, ultrasound energy) toward the receiver-stimulator 110, which can receive the energy and deliver one or more electrical pulses (e.g., stimulation pulses, pacing pulses) to the heart 102.

In some embodiments, the system 100 can further include a programmer 130 in operable communication with the controller-transmitter 120. The programmer 130 can be positioned outside the body 104 and operable to program (e.g., by a physician) various parameters of the controller-transmitter 120 and/or to receive diagnostic information from the controller-transmitter 120. In some embodiments, the system 100 can further include a co-implant device 132 (e.g., an implantable cardioverter defibrillator (ICD) or pacemaker) coupled to pacing leads 134 for delivering stimulation pulses to one or more portions of the heart 102 other than the area stimulated by the receiver-stimulator 110. In other embodiments, the co-implant device 132 can be a leadless pacemaker which is implanted directly into the heart to eliminate the need for separate pacing leads 134. The co-implant device 132 and the controller-transmitter 120 can operate in tandem and deliver stimulation signals to the heart 102 to cause a synchronized heartbeat. In some embodiments, the controller-transmitter 120 can receive signals (e.g., electrocardiogram signals) from the heart 102 to determine information related to the heart 102, such as a heart rate, heart rhythm, including the output of the pacing leads 134 located in the heart 102. In some embodiments, as described in greater detail below with reference to Figures 2-8, the controller-transmitter 120 can alternatively or additionally be configured to receive information (e.g., diagnostic signals) from the receiver-stimulator 110. The received signals can be used to adjust the ultrasound energy signals delivered to the receiver-stimulator 110.

The receiver-stimulator 110, the controller-transmitter 120, and/or the programmer 130 can include a machine-readable (e.g., computer-readable) or controller-readable medium containing instructions for generating, transmitting, and/or receiving suitable signals (e.g., stimulation signals, diagnostic signals). The receiver-stimulator 110, the controller-transmitter 120, and/or the programmer 130 can include one or more processor(s), memory unit(s), and/or input/output device(s). Accordingly, the process of providing stimulation signals and/or executing other associated functions can be performed by computer-executable instructions contained by, on, or in computer-readable media located at the receiver-stimulator 110, the controller-transmitter 120, and/or the programmer 130. Further, the receiver-stimulator 110, the controller-transmitter 120, and/or the programmer 130 can include dedicated hardware, firmware, and/or software for executing computer-executable instructions that, when executed, perform any one or more methods, processes, and/or sub-processes described herein. The dedicated hardware, firmware, and/or software also serve as "means for" performing the methods, processes, and/or sub-processes described herein.

In some embodiments, the system 100 can include several features generally similar or identical to those of the leadless tissue stimulation systems disclosed in (i) U.S. Patent 7,610,092, filed December 21, 2005, and titled "LEADLESS TISSUE STIMULATION SYSTEMS AND METHODS," (ii) U.S. Patent 8,315,701, filed September 4, 2009, and titled "LEADLESS TISSUE STIMULATION SYSTEMS AND METHODS," and/or (iii) U.S. Patent 8,718,773, filed May 23, 2007, and titled "OPTIMIZING ENERGY TRANSMISSION IN A LEADLESS TISSUE STIMULATION SYSTEM."

Figure 2 is a schematic diagram of a leadless tissue stimulation system 200 ("system 200") configured in accordance with embodiments of the present technology. The system 200 can include several features generally similar or identical to the features of the system 100 described in detail above with reference to Figure 1. For example, the system 200 can include a receiver-stimulator 210 in operable communication with a controller-transmitter 220.

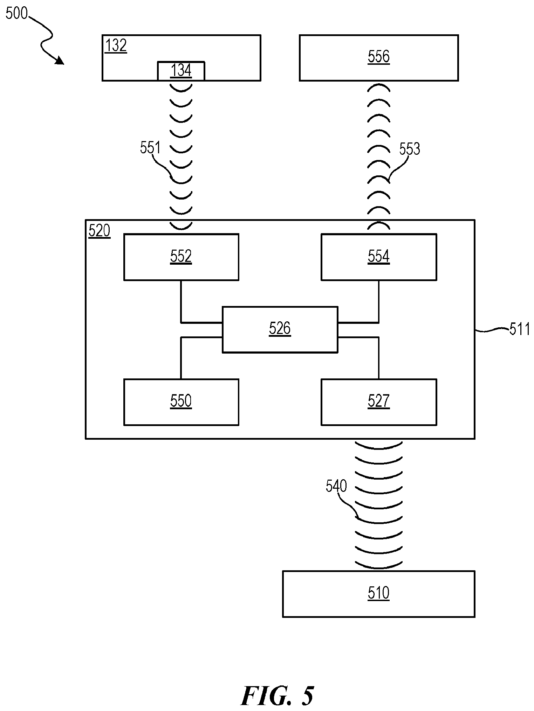

In the illustrated embodiment, the controller-transmitter 220 includes a signal conditioning and processing circuit 225, a controller 226 (e.g., control and timing circuitry, a communications module, a voltage regulator), a first communication circuit 227, second communication circuit 228, and a receiving circuit 229. In some embodiments, the signal conditioning and processing circuit 225 can receive signals from the receiving circuit 229 and/or signals from one more additional sources 221 and condition and/or process these signals before passing them to the controller 226. In some embodiments, the additional sources 221 can include an external programmer, such as the programmer 130 of Figure 1. Moreover, as described in detail below with reference to Figure 5, in some embodiments the signals from the additional source(s) 221 can include signals (e.g., pacing signals) from a co-implant device and/or physiological signals from one or more physiological sensors in and/or carried by the controller-transmitter 220. The controller 226 can use the information received from the signal conditioning and processing circuit 225 to generate (i) first control signals for the first communication circuit 227 to produce a first signal 240 for transmission to the receiver-stimulator 210 and/or (ii) second control signals for the second communication circuit 228 to produce a second signal 242 for transmission to the receiver-stimulator 210.

In some embodiments, the first signal 240 can be an ultrasound energy signal (e.g., an ultrasound energy transmission) having a particular frequency range such as for example, 500 kHz to 10 MHz, 800 kHz to 2 MHz, or 900 kHz to 1 MHz (e.g., about 921 kHz). The controller-transmitter 220 can transmit the first signal 240 as a focused beam of wireless energy (e.g., ultrasound energy). Accordingly, the first communication circuit 227 can comprise one or more transducers, signal generators, amplifiers, and the like for generating the first signal 240 and directing the first signal 240 toward the receiver-stimulator 210. In some embodiments, the second signal 242 is an electromagnetic signal having a frequency greater than a frequency of the first signal 240. For example, the second signal 242 can be a radiofrequency (RF) signal having a frequency of greater than 1 MHz, greater than 10 MHz, greater than 100 MHz, greater than 400 MHz, or greater. In a particular embodiment, the second signal 242 has a frequency of about 402-405 MHz. Accordingly, the second communication circuit 228 can comprise one or more signal generators, amplifiers, antennas, and the like for generating the second signal 242 and directing the second signal 242 toward the receiver-stimulator 210. Thus, in some aspects of the present technology the system 200 is a dualfrequency system operating at both an acoustic (e.g., ultrasound) frequency and a radiofrequency.

In general, the receiver-stimulator 210 includes a housing 211 containing and/or carrying various electronic components, circuit components, circuit blocks, functional blocks, and the like, that collectively enable the receiver-stimulator 210 to (i) receive the first signal 240 and the second signal 242 from the controller-transmitter 220, (ii) transmit a third signal 244 to the controller-transmitter 220, and (iii) deliver one or more electrical pulses (e.g., pacing pulses, stimulation pulses) to tissue of a patient (e.g., the heart 102 shown in Figure 1). More specifically, in the illustrated embodiment the receiver-stimulator 210 includes a transducer-pacing circuit 212 that receives the first signal 240 and converts the first signal 240 into an electrical pacing pulse for delivery via one or more electrodes 213 (e.g., a pair of stimulation electrodes). In some embodiments, the transducer-pacing circuit 212 includes one or more piezoelectric elements (e.g., including crystal, ceramic, and/or other materials) that accumulate electrical charge in response to receiving the first signal 240, and circuitry (e.g., detector circuitry, envelope detector circuitry, rectifier circuitry, filtering circuitry, voltage-limiting circuitry) for producing a voltage pulse with an amplitude and length proportional to the first signal 240. The transducer-pacing circuit 212 can deliver the voltage pulse to the electrodes 213, which can project past and/or be incorporated into an outer surface of the housing 211 so as to contact the tissue of the patient when the receiver-stimulator 210 is in implanted therein. In some embodiments, the housing 211 comprises a hermetically sealed case of biologically compatible material.

In the illustrated embodiment, the receiver-stimulator 210 further includes a first antenna 214 that can receive the second signal 242 from the controller-transmitter 220, convert the second signal 242 to an electrical signal, and pass the electric signal to an energy-harvesting circuit 215. In the illustrated embodiment, the receiver-stimulator 210 further includes a communication circuit 217 and a physiological sensor 218 (e.g., a cardiac sensor) electrically coupled to a controller 216 (e.g., a processor, a voltage regulator). The energy-harvesting circuit 215 produces power (e.g., harvests energy) from the second signal 242 and passes that power to the controller 216. In some embodiments, the energy-harvesting circuit 215, the controller 216, the communication circuit 217, and/or another component (e.g., a capacitor, a battery) of the receiver-stimulator 210 can store the energy produced by the energy-harvesting circuit 215. The physiological sensor 218 can sense one or more intrinsic cardiac parameters of the heart of the patient (e.g., the heart 102 of Figure 1) at which the receiver-stimulator 210 is implanted. For example, the physiological sensor 218 can comprise an intracardiac electrogram (IEGM) sensor and, in some embodiments, can measure the IEGM at the electrodes 213. In some embodiments, the physiological sensor 218 can additionally or alternatively include a transducer (e.g., a low frequency transducer, a microphone) that detects one or more cardiac sounds, such as sounds corresponding to the operation of a heart valve (e.g., an aortic valve, a mitral valve). Similarly, the physiological sensor 218 can detect the onset of certain cardiac events such as diastole, systole, and so on.

The communication circuit 217 can be electrically coupled to a second antenna 219 and can generate and/or pass electrical signals to the second antenna 219 for transmission to the controller-transmitter 220 (e.g., to the receiving circuit 229) as the third signal 244. In some embodiments, the communication circuit 217 performs pulse width modulation of signals from the controller 216 (and/or other components of the receiver-stimulator 210) at radiofrequency. In some embodiments, the third signal 244 has a frequency that is the same as or generally similar to the frequency of the second signal 242 (e.g., a RF-signal having a frequency of about 402-405 MHz). The communication circuit 217 and the second antenna 219 can be configured (e.g., shaped, positioned, tuned) to transmit the third signal 244 toward the controller-transmitter 220 as a focused beam of wireless energy. In some embodiments, the first and second antennas 214, 219 comprise multiple windings of a metal wire material (e.g., gold).

In some embodiments, the controller 216 can be an application-specific integrated circuit (ASIC) or other type of controller configured to at least partially control operation of the various electronic components of the receiver-stimulator 210. For example, the controller 216 can receive the power produced by the energy-harvesting circuit 215 and the physiological parameters detected by the physiological sensor 218 and send control signals to the communication circuit 217 to produce the third signal 244 for transmission to the controller-transmitter 220 including encoded information about the physiological parameters. That is, the controller 216 can utilize the power transferred via the second signal 242 to generate and transmit the third signal 244 (e.g., via the communication circuit 217 and second antenna 219) back to the controller-transmitter 220 including information about detected physiological parameters in real-time or near real-time. The receiving circuit 229 of the controller-transmitter 220 can include an antenna and/or other components for receiving and decoding the third signal 244 before passing the third signal 244 to, for example, the signal conditioning and processing circuit 225 and the controller 226. In some embodiments, the powered signal can additionally or alternatively include information about a status of the receiver-stimulator 210, an efficiency of pacing stimulation via the electrodes 213, and/or other information detected by the receiver-stimulator 210. The controller-transmitter 220 can utilize the information received via the third signal 244 to modify the first signal 240-such as a timing, amplitude, pulse length, and/or other characteristic of the first signal 240-to thereby control the electrical stimulation (e.g., pacing stimulation) output by the electrodes 213.

The power produced by the energy-harvesting circuit 215 can alternatively or additionally be used to provide stimulation pulses to the tissue of the patient via the electrodes 213. For example, in some embodiments the controller 216 can combine and/or manage the power received from the first signal 240 and the second signal 242 and provide an appropriate stimulation pulse or plurality of stimulation pulses to the electrodes 213 (e.g., via the transducer-pacing circuit 212). In some embodiments, power from the second signal 242 can be used as a backup power source for powering the electrodes 213 in the event the first signal 240 is lost. In some embodiments, power from the second signal 242 can be used to power the physiological sensor 218 and/or other electronic components of the receiver-stimulator 210.

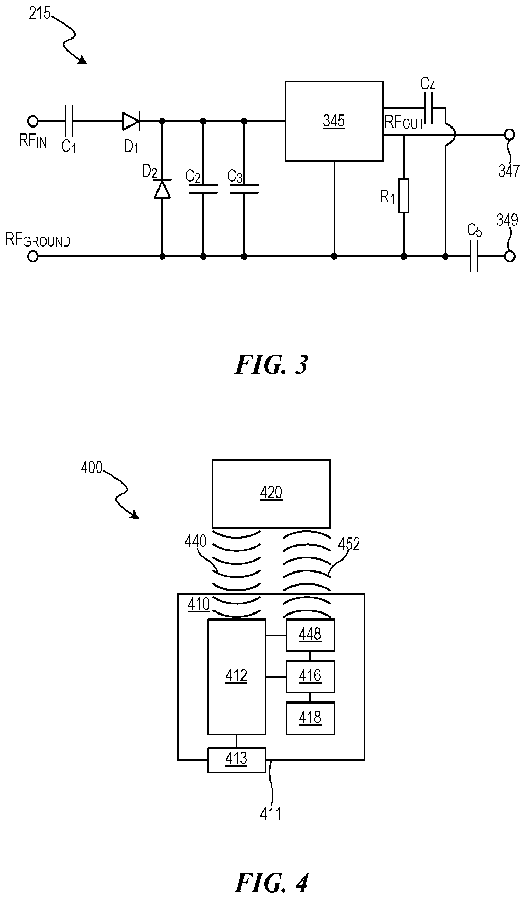

Figure 3 illustrates the energy-harvesting circuit 215 of the receiver-stimulator 210 shown in Figure 2 configured in accordance with embodiments of the present technology. Referring to Figures 2 and 3 together, the energy-harvesting circuit 215 can harvest energy from the second signal 242 (e.g., a radiofrequency signal) transmitted from the controller-transmitter 220 to the receiver-stimulator 210. After receiving the second signal 242 through the first antenna 214, the first antenna 214 passes a radiofrequency input signal RF_{IN} to the energy-harvesting circuit 215. As shown in Figure 3, the energy-harvesting circuit 215 includes electrical connectors for receiving the input signal RF_{IN} and a ground signal RF_{GROUND}. The input signal RF_{IN} can be passed through a first diode D₁ and a second diode D₂ to rectify the input signal RF_{IN}. In some embodiments, the first and second diodes D₁ and D₂ can be Schottky diodes, which have a low forward voltage for energy efficiency. In the illustrated embodiment, the energy-harvesting circuit 215 further includes a network of capacitors C (including individually identified first through fifth capacitors C₁-C₅, respectively). The first through third capacitors C₁-C₃ can be connected across the input signal RF_{IN} to provide a selected capacitance for signal conditioning before the rectified and conditioned input signal RF_{IN} is input into a charge pump 345. In some embodiments, the charge pump 345 can be of the type sold by Seiko Instruments Inc., under the product model "S882Z." In other embodiments, the charge pump 345 can be another type of charge pump and/or can be integrated into the controller 216 (Figure 2).

In the illustrated embodiment, the charge pump outputs an output signal RF_{OUT} that can be further conditioned via a resistor R1 (e.g., a high value resistor (HVR)), the fourth capacitor C₄, and/or the fifth capacitor C₅. Referring to Figures 2 and 3 together, the conditioned output signal RF_{OUT} can be output to the controller 216 (e.g., a voltage regulation circuit of the controller 216) via an anode 347 and a cathode 349. In other embodiments, the energy-harvesting circuit 215 can include more or fewer electronic components suitable for harvesting the energy from the second signal 242 and outputting the energy (e.g., a voltage) to the controller 216 and/or another component of the receiver-stimulator 210.

Figure 4 is a schematic diagram of a tissue stimulation system 400 ("system 400") configured in accordance with embodiments of the present technology. The system 400 can include several features generally similar or identical to the features of the systems 100 and/or 200 described in detail above with reference to Figures 1-3. For example, the system 400 can include a receiver-stimulator 410 in operable communication with a controller-transmitter 420. Likewise, in the illustrated embodiment the receiver-stimulator 410 includes a housing 411 containing and/or carrying a transducer-pacing circuit 412, stimulation electrodes 413, a controller 416 (e.g., a voltage-regulating ASIC), and a physiological sensor 418. The transducer-pacing circuit 412 can receive a first signal 440 (e.g., an acoustic ultrasound signal) from the controller-transmitter 420 and convert the first signal 440 into an electrical pacing pulse for delivery via one or more of the electrodes 413.

In the illustrated embodiment, the receiver-stimulator 410 further includes a communication circuit 448 electrically coupled to the transducer-pacing circuit 412 and/or the controller 416. The communication circuit 448 can receive a portion of the first signal 440 and encode and/or modulate the portion of the first signal 440 with information from the physiological sensor 418 and/or status information about the receiver-stimulator 410 for transmission back to the controller-transmitter 420 as a second signal 452. In some embodiments, the communication circuit 448 is configured to encode the information into the second signal 452 using pulse position and/or pulse width codes. In some embodiments, the first and second signals 440, 452 can be acoustic signals (e.g., ultrasound signals) having the same or substantially similar frequency such as, for example, between about 500 kHz to 10 MHz, 800 kHz to 2 MHz, or 900 kHz to 1 MHz (e.g., about 921 kHz). Therefore, the communication circuit 448 can include one or more transducer elements (e.g., piezoelectric elements), antennas, and/or modulation components configured to generate the second signal 452 and direct the second signal 452 toward the controller-transmitter 420 (e.g., an ultrasound receiver therein) as a focused beam of ultrasound energy. Accordingly, in some aspects of the present technology the system 400 is a single-frequency system operating to both receive the first signal 440 at and transmit the second signal 452 from the receiver-stimulator 410 at the same or substantially similar frequency.

In other embodiments, the communication circuit 448 can comprise a speaker or piezoelectric beeper that can transmit the second signal 452 as audible sounds toward the controller-transmitter 420. In such embodiments, the controller-transmitter 420 can include a microphone for receiving the second signal 452 and converting the second signal 452 to an electric signal.

Figure 5 is a is a schematic diagram of a tissue stimulation system 500 ("system 500") configured in accordance with embodiments of the present technology. The system 500 can include several features generally similar or identical to the features of the systems 100, 200, and/or 400 described in detail above with reference to Figures 1-4. For example, the system 500 can include a receiver-stimulator 510 in operable communication with a controller-transmitter 520. The controller-transmitter 520 can include a controller 526 electrically coupled to a first communication circuit 527 and configured to generate first control signals for the first communication circuit 527 to produce a first signal 540 (e.g., an acoustic ultrasound signal) for transmission to the receiver-stimulator 510. The receiver-stimulator 510 can receive the first signal 540 and output an electrical stimulation pattern based on the first signal 540.

In the illustrated embodiment, the controller-transmitter 520 further includes a physiological sensor 550 that can, for example, sense one or more intrinsic cardiac parameters of a heart of a patient (e.g., the heart 102 of Figure 1). In some embodiments, the physiological sensor 550 can include one or more electrodes positioned on a casing 511 of the controller-transmitter 520 (e.g., on an exterior or outer surface of the casing 511 facing the heart) for sensing a far-field electrogram (EGM), an impedance, plethysmography information, and/or hemodynamic activity. In additional embodiments, the physiological sensor 550 can be an optical sensor (e.g., a photoplethysmography (PPG) optical sensor), a microphone configured to detect one or more cardiac sounds such as mitral and/or aortic valve heart sounds, and/or another type of sensor. After detecting the one or more physiological parameters, the physiological sensor 550 can pass a signal representative of the parameters to the controller 526 and/or another electronic component of the controller-transmitter 520.

In the illustrated embodiment, the controller-transmitter 520 further includes a receiving circuit 552 that can receive a second signal 551 from a co-implant device (e.g., the co-implant device 132 of Figure 1). The second signal 551 can include instructions for programming parameters (e.g., operational parameters) of the controller-transmitter 520 to trigger a specific output of the first signal 540. In some embodiments, the second signal 551 is a radiofrequency signal and the receiving circuit 552 can include an antenna and/or other components for receiving and decoding the second signal 551. Accordingly, the second signal 551 can be a wireless radiofrequency communication signal. In other embodiments, the second signal 551 can be an electrical signal (e.g., an electrical pulse) generated by the pacing leads 134 of the co-implant device 132 and/or other electrodes of the co-implant device 132 such as, for example, dedicated communication electrodes positioned on an external surface of the co-implant device 132. In other embodiments, the controller-transmitter 520 can be communicatively coupled to the co-implant device 132 via a different wireless or wired communication link. After receiving, decoding, and/or processing the second signal 551, the receiving circuit 552 can pass the second signal 551 to the controller 526 and/or another electronic component of the controller-transmitter 520.

The controller 526 is electrically coupled to the physiological sensor 550 and the receiving circuit 552 and can generate the first control signals for generating the first signal 540 based at least in part on (i) the physiological parameters detected by the physiological sensor 550 and/or (ii) the instructions transmitted by the co-implant device 132. That is, the controller 526 can cause the first communication circuit 527 to generate and transmit the first signal 540 in accordance with sensed physiological parameters of the patient and/or instructions from the co-implant device 132 to, for example, cause the receiver-stimulator 510 to produce a specific stimulation output. In some embodiments, the controller-transmitter 520 can be configured as a "slave" to the "master" co-implant device 132 such that the parameters (e.g., amplitude, timing, pulse characteristics) of the first signal 540 are driven only or substantially only by the instructions of the co-implant device 132 communicated via the second signal 551.

In the illustrated embodiment, the controller-transmitter 520 further includes a second communication circuit 554 operably coupled to the controller 526. The controller 526 can generate second control signals for the second communication circuit 554 to produce a third signal 553 for transmission to an external device 556, such as a smartphone, tablet, computer, or other electronic device. In some embodiments, the third signal 553 is a Bluetooth signal (e.g., a Bluetooth Low Energy (BLE) signal) that communicatively couples the controller-transmitter 520 to the external device 556. Accordingly, the second communication circuit 554 can comprise a Bluetooth chip including a Bluetooth antenna, amplifier, and/or other electronic components. In some embodiments, the controller 526 can operate the second communication circuit 554 to communicate data from the physiological sensor 550, data from the co-implant device 132, status information, and/or other information to the external device 556 via the third signal 553. In some embodiments, the external device 556 can be a device of a physician of the patient such that information can be automatically communicated from the system 500 to the physician without the intervention of the patient.

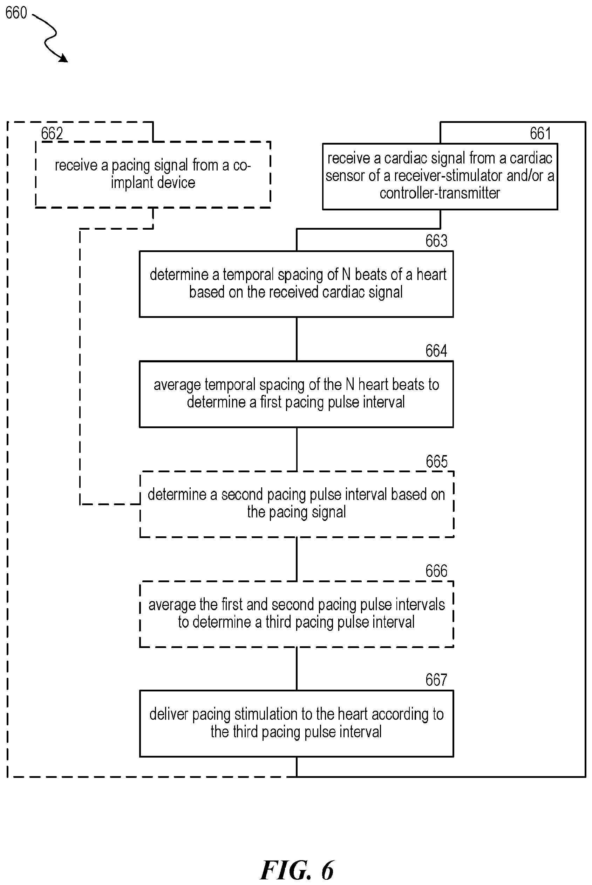

Figure 6 is a flow diagram of a process or method 660 for using a tissue stimulation system to generate and deliver electrical stimulation (e.g., electrical pacing pulses) to the heart of a patient in accordance with embodiments of the present technology. Although some features of method 660 are described in the context of the embodiments described in detail with reference to Figures 1-5, one skilled in the art will readily understand that the method 660 can be carried out using other suitable systems and/or devices described herein.

At block 661, the method 660 includes receiving a cardiac signal from a cardiac sensor of a receiver-stimulator implanted at the heart and/or a controller-transmitter implanted in the patient remote from the heart. For example, in some embodiments the controller 226 of the controller-transmitter 220 can wirelessly receive the cardiac signal 244 from the physiological sensor 218 of the receiver-stimulator 210, and/or receive the cardiac signal 452 from the physiological sensor 418 of the receiver-stimulator 410. In other embodiments, the physiological sensor 550 of the receiver-stimulator 510 can directly detect the cardiac signal. The cardiac signal can include EGM information, plethysmography information, audio information, and/or other cardiac information.

Optionally, at block 662, the method 660 can include receiving a pacing signal from a co-implant device. For example, in some embodiments the controller-transmitter 520 can directly receive the pacing signal 551 from the co-implant device 132 over a wireless communication link or can detect the pacing signal via, for example, one or more electrodes of the controller-transmitter 520.

At block 663, the method 660 includes determining, based on the received cardiac signal, a temporal spacing of a predetermined number (N) beats of the heart. In general, this determination is based on the specific type of cardiac signal received from the cardiac sensor. For example, where the cardiac signal is an ECG signal (e.g., recorded at the controller-transmitter or recorded at the receiver-stimulator and wirelessly transmitted to controller-transmitter), the block 663 can include detecting a temporal spacing between one or more of a P-wave (e.g., representing atrial depolarization), a QRS-segment (e.g., representing ventricular depolarization), a T-wave (e.g., representing ventricular repolarization), and/or other features in the ECG waveform in successive heart beats. Similarly, where the cardiac signal is a plethysmography signal, the block 663 can include detecting a temporal spacing between spikes in the plethysmography waveform representing blood volume (e.g., a minimum or maximum blood volume) in successive heart beats. Likewise, where the cardiac signal comprises audio information about heart function (e.g., heart valve opening and/or closing), the block 663 can include detecting a temporal spacing between features in the audio waveform representing the opening and closing of heart valves, the contraction and expansion of the atria and/or ventricles, and so on, in successive heart beats. In some embodiments, the cardiac signal can be processed (e.g., filtered) to better facilitate determination of the temporal spacing between heart beats.

At block 664 the method 660 includes averaging the temporal spacing of the N heart beats to determine a first pacing pulse interval by, for example, dividing the temporal spacing by N. For example, where the temporal spacing between 10 heart beats (i.e., N=10) is determined at block 663, the block 664 can include dividing the total time elapsed between the first detected heart beat and the tenth detected heart beat by 10 to determine the first pacing pulse interval. In some embodiments, the number of heart beats N can be two, between two to ten, ten, or greater than ten.

At block 665, the method 660 can optionally include determining a second pacing pulse interval based on the pacing signal optionally received at block 662. In general, this determination is based on the specific type of pacing signal received from the cardiac sensor. For example, where the co-implant device 132 directly transmits information (e.g., the second signal 551) to the controller-transmitter 520 (e.g., over a RF communication link), the transmitted information can include the second pacing pulse interval. Where the controller-transmitter directly detects the pacing signal of the co-implant device 132 (e.g., via one or more electrodes), determining the second pacing pulse interval can include determining a temporal spacing between features (e.g., electrical spikes) representing successive heart beats and averaging over the number of heart beats (e.g., similar to the methodology of blocks 663 and 664).

At block 666, the method 660 can optionally include averaging the first and second pacing pulse intervals to determine a third pacing pulse interval. At block 667, the method can include delivering pacing stimulation to the heart according to the third pacing pulse interval, or according to the first pacing pulse interval if the second pacing pulse interval is not detected (i.e., in the omission of optional blocks 662, 665, and/or 666). For example, the controller 226 can send control signals to the first communication circuit 227 to produce the first signal 240 according the determined third pacing pulse interval (e.g., with the third pacing pulse interval encoded therein). The transducer-pacing circuit 212 can receive the first signal 240 and convert the first signal 240 to an electrical pacing output that is delivered via the electrodes 213 according to the third pacing interval.

Accordingly, in some aspects of the present technology the method 660 can help ensure that the electrodes 213 deliver pacing stimulation at specifically selected times relative to the heart beat of the patient. For example, in some embodiments the pacing stimulation can be reliably applied slightly ahead of ventricular depolarization (e.g., the QRS-segment of the ECG). Moreover, the method 660 is not dependent upon the morphologies or amplitudes of the cardiac signal, but rather on the temporal characteristics of the cardiac signal.

In some embodiments, the method 660 can return to blocks 661 and/or 662. For example, the method 660 can include delivering pacing stimulation (the block 667) for a predetermined amount of time and/or a predetermined number of heart beats before the method 660 returns to blocks 661 and/or 662 to recalibrate the pacing pulse interval.

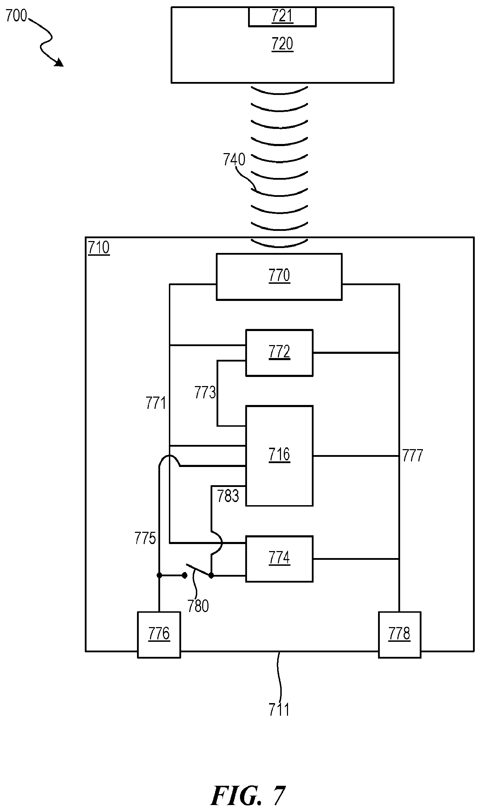

Figure 7 is a partially schematic diagram of a tissue stimulation system 700 ("system 700") configured in accordance with embodiments of the present technology. The system 700 can include several features generally similar or identical to the features of the systems 100, 200, 400, and/or 500 described in detail above with reference to Figures 1-5. For example, the system 700 can include a receiver-stimulator 710 in operable communication with a controller-transmitter 720. The controller-transmitter 720 can generate and transmit a signal 740 (e.g., an acoustic ultrasound signal) to the receiver-stimulator 710 including electrical stimulation parameters (e.g., pacing parameters provided by an external programmer, such as the programmer 130 of Figure 1).

In some embodiments, the controller-transmitter 720 can modulate the signal 740 to include the electrical stimulation parameters. For example, the controller-transmitter 720 can pulse modulate the signal 740 such that a duration of transmission bursts or a time between subsequent transmission bursts encodes the electrical stimulation parameters. In some embodiments, the electrical stimulation parameters can include a minimum pacing rate, a threshold detection level for sensing cardiac depolarization, a pacing pulse output voltage, and/or the like. The receiver-stimulator 710 can receive the signal 740 and convert the signal 740 into electrical power for stimulating tissue according to the electrical stimulation parameters. In some embodiments, the controller-transmitter 720 includes a sensor 721 (e.g., one or more electrodes) for detecting an electrical output of the receiver-stimulator 710.

More specifically, in the illustrated embodiment the receiver-stimulator 710 includes a housing 711 containing and/or carrying a plurality of electronic components including a controller 716, a transducer circuit 770, a first storage unit 772, a second storage unit 774, a cathode 776, and an anode 778 (the cathode 776 and the anode 778 can collectively be referred to as "electrodes"). The cathode 776 and the anode 778 can project past and/or be incorporated into an outer surface of the housing 711 and are configured to be electrically coupled to tissue (e.g., cardiac tissue) of a patient for delivering electrical stimulation energy and/or sensing electrical signals through the tissue. The transducer circuit 770 (e.g., an energy-harvesting circuit) can receive the signal 740, convert the signal 740 to electrical power, and pass the electrical power to the first storage unit 772 and the second storage unit 774 via a first electrical line 771 (e.g., a negative output of the transducer circuit 770). That is, the transducer circuit 770 can harvest the energy from the signal 740 to charge the first and second storage units 772, 774. The first and second storage units 772, 774 can be capacitors, super-capacitors, rechargeable batteries, and/or other components that can store electrical energy. In the illustrated embodiment, the first storage unit 772 powers the controller 716 via a second electrical line 773.

The controller 716 can receive inputs from (i) the transducer circuit 770 via the first electrical line 771, (ii) the cathode 776 via a third electrical line 775 (e.g., a cathode bus), and (iii) the anode 778 via a fourth electrical line 777. A fourth electrical line 777 from the anode 778 can provide a ground reference for the controller 716 and/or other components of the receiver-stimulator 710. In the illustrated embodiment, the second storage unit 774 is electrically coupled to the cathode 776 via a switch 780 (e.g., and the third electrical line 775 and/or one more additional electrical lines), and provides electrical power to the cathode 776 when the switch 780 is closed. In other embodiments, the switch 780 can be positioned between the second storage unit 774 and the anode 778, and the cathode 776 can be electrically coupled to the first electrical line 771.

In some embodiments, the controller 716 can amplify input signals from the cathode 776 and the anode 778 and apply a detection algorithm to detect certain cardiac events. For example, the controller 716 can apply a threshold-level detector or other suitable algorithm for sensing cardiac depolarization events. The controller 716 can further determine when to electrically stimulate the tissue (e.g., pace the tissue) via the cathode 776 and the anode 778 based on (i) the timing of a sensed cardiac depolarization event (and/or another cardiac event) and/or (ii) the electrical stimulation parameters encoded in the signal 740. To apply the electrical stimulation energy, the controller 716 can output a control signal over a sixth electrical line 783 to (i) close the switch 780 such that the second storage unit 774 delivers the electrical stimulation energy to the cathode 776 and then (ii) open the switch 780 to achieve a desire pacing pulse width.

In some embodiments, the receiver-stimulator 710 can send a communication signal to the controller-transmitter 720 to indicate that the first storage unit 772 and/or the second storage unit 774 are fully charged. For example, the receiver-stimulator 710 can deliver an electrical energy pulse (e.g., a short duration, sub-stimulation pulse) to the cathode 776 that can be detected by the controller-transmitter 720 (e.g., the sensor 721 of the controller-transmitter 710). In other embodiments, the receiver-stimulator 710 can send a radiofrequency or ultrasound signal to the controller-transmitter 720 as described in detail above, for example, with reference to Figures 2-4. After receiving the communication signal, the controller-transmitter 720 can terminate the transmission of the signal 740 for a predetermined amount of time.

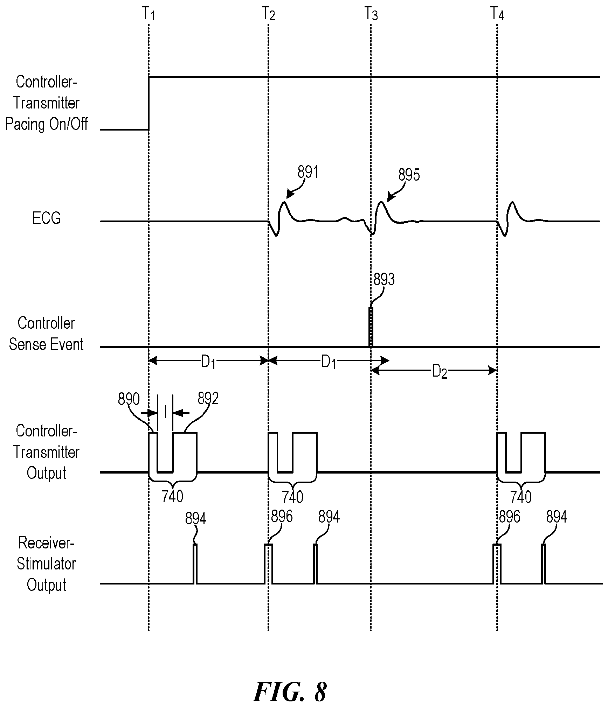

Figure 8 illustrates an example control scheme and timing sequence for the system 700 of Figure 7 in a pacemaker application (e.g., a bradycardia pacemaker application) with the receiver-stimulator 710 (Figure 7) implanted at a heart of a patient in accordance with embodiments of the present technology. Figure 8 depicts the evolution over time of (i) the on/off state of the system 700 ("Controller-Transmitter Pacing On/Off"), (ii) the ECG of a patient ("ECG") which is representative of an EMG detected via the cathode 776 and/or the anode 778, (iii) the sensing of a cardiac event by the controller 716 ("Controller Sense Event"), (iv) the acoustic output signal 740 of the controller-transmitter 720 ("Controller-Transmitter Output"), and (v) the electrical output of the receiver-stimulator 710 ("Receiver-Stimulator Output").

In the illustrated embodiment, the system 700 is enabled for a pacing mode at a first time T₁ ("turned on"). When the pacing mode is enabled, the controller-transmitter 720 can output the signal 740 including a short first transmission burst 890 and a longer second transmission burst 892 separated by a time interval I. In some embodiments, the time interval I encodes a maximum allowable cardiac interval D₁ between heart beats for the pacemaker application. The transducer circuit 770 of the receiver-stimulator 710 can receive the second transmission burst 892 and convert the acoustic energy to electrical energy for charging the first and second storage units 772, 774. In some embodiments, the receiver-stimulator 710 can determine the length of the second transmission burst 892 by, for example, determining when the first and second storage units 772, 774 are sufficiently (e.g., fully) charged and outputting an output signal 894 (e.g., a communication signal, a communication pulse) via the cathode 776 and/or anode 778 indicating that charging is sufficient. The controller-transmitter 720 can detect the output signal 894 (e.g., via the sensor 721) and cease transmission of the signal 740 upon detection. In some embodiments, the output signal 894 is a short-duration electrical signal that is not configured (e.g., has a low enough amplitude and/or duration) to stimulate the heart of the patient. In some aspects of the present technology, this arrangement can ensure that the receiver-stimulator 710 is optimally charged (e.g., to provide a specified output pacing voltage) regardless of the acoustic transfer path between the controller-transmitter 720 and the receiver-stimulator 710 and any changes in the acoustic transfer path over time (e.g., regardless of any variability of the acoustic transfer path over different heart beats).

After receiving the signal 740, the controller 716 can set a timer for the specified cardiac interval D₁ while monitoring the heart of the patient to sense a specified cardiac event, such as a cardiac depolarization event in EMG data sensed via the cathode 776 and/or the anode 778. If the controller 716 does not sense the specified cardiac event after the interval D₁, the controller 716 can send the control signal to close the switch 780 to deliver all or a portion of the electrical energy stored in the second storage unit 774 to the cathode 776 and/or the anode 778 to stimulate (e.g., pace) the heart of the patient. After a time period equivalent to the desired pacing pulse width, the controller 716 can then send a signal to open the switch 780. In the illustrated embodiment, for example, the receiver-stimulator 710 delivers a pacing pulse 896 at a second time T₂ after the cardiac interval D₁ has elapsed after the first time T₁. The pacing pulse 896 can stimulate the heart to beat, as represented in the sensed ECG by an induced wave 891 following the time T₂. After the receiver-stimulator 710 delivers the pacing pulse 896, the controller-transmitter 720 can detect the delivered pacing pulse 896 and recharge the receiver-stimulator 710 by again transmitting the signal 740 to charge the first and second storage units 772, 774, and the receiver-stimulator 710 can cease the transmission of the signal 740 by again outputting the output signal 894 when the first and second storage units 772, 774 are sufficiently charged,

If the controller 716 does sense the specified cardiac event during the interval D₁, the controller 716 can maintain the switch 780 in the open position such that no stimulation is applied to the heart. For example, in the illustrated embodiment the controller 716 senses the specified cardiac event (e.g., as represented by an event marker 893 corresponding to an intrinsic cardiac activity 895) at a third time T₃ during the interval 01 after the second time T₂. Accordingly, the receiver-stimulator 710 does not output the pacing pulse 896 and the controller-transmitter 720 does not retransmit the signal 740. At this time, the controller 716 can update the initial interval D₁ to a second interval D₂ based on the detected event marker 893. The interval D₁ can be the same or different than the interval D₂.

The continued monitoring of cardiac events, charging of the receiver-stimulator 710, and delivery of the pacing pulse 896 can continue for as long as the pacing mode of the controller-transmitter 710 is enabled. For example, in the illustrated embodiment the receiver-stimulator 710 delivers another pacing pulse 896 at a fourth time T₄ after the cardiac interval D₂ has elapsed after the third time T₃. In some embodiments, the cardiac interval and/or parameters of the pacing pulse 896 can be modified by the controller-transmitter 720 in different transmissions of the signal 740 (e.g., by changing the initial interval D₁ to a second interval D₂). Moreover, additional information and/or parameters can be encoded in the signal 740 by, for example, breaking the signal 740 up into additional bursts. In such embodiments, a delay between each burst can correspond to a different parameter.

The above detailed description of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, although steps are presented in a given order, alternative embodiments can perform steps in a different order. Likewise, the various electronic components and functions can be separated into more or fewer electronic circuit elements and/or functional blocks. The various components and/or functionalities of the embodiments described herein can also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms can also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications can be made without deviating from the technology. Further, while advantages associated with some embodiments of the technology have been described in the context of those embodiments, other embodiments can also exhibit such advantages, and not all embodiments need necessarily exhibit such advantages to fall within the scope of the technology. Accordingly, the disclosure and associated technology can encompass other embodiments not expressly shown or described herein.

## Claims

1. A system (200) for pacing cardiac tissue of a patient, comprising:
a controller-transmitter (220) including a first circuit (227) and a second circuit (229), wherein the first circuit is configured to transmit an acoustic signal (240), and wherein the second circuit (229) is configured to receive a radiofrequency signal (244); and
a receiver-stimulator (210) configured to be implanted in the patient proximate the cardiac tissue, wherein the receiver-stimulator (210) includes-
an electrode (213);
a third circuit (212) operably coupled to the electrode and configured to receive at least a portion of the acoustic signal (240) and convert the acoustic signal (240) to electrical energy for delivery to the cardiac tissue via the electrode (213) to electrically pace the cardiac tissue; and
a fourth circuit (217) configured to transmit the radiofrequency signal (244) to the second circuit (229) of the controller-transmitter (220);
wherein the controller-transmitter (220) is configured to modify the acoustic signal based on the radiofrequency signal (244) to control the electrical energy delivered to the cardiac tissue via the electrode (213) to control the electrical pacing of the cardiac tissue.

2. The system of claim 1 wherein the receiver-stimulator (210) further comprises a physiological sensor (218) configured to sense a physiological parameter of the patient, and wherein the radiofrequency signal (244) includes information about the sensed physiological parameter.

3. The system of claim 2 wherein the sensed physiological parameter is an intracardiac electrogram (IEGM) or wherein the sensed physiological parameter is a cardiac sound.

4. The system of claim 2 wherein the controller-transmitter further comprises a controller electrically coupled to the first circuit (227) and the second circuit (229), wherein the controller is configured to control the first circuit to transmit the acoustic signal based at least in part on the sensed physiological parameter.

5. The system of claim 1 wherein the radiofrequency signal (244) includes information about a status of the receiver-stimulator (210).

6. The system of claim 1 wherein the radiofrequency signal (244) includes information about an efficiency of delivery of the electrical energy to the cardiac tissue.

7. The system of claim 1 wherein
the radiofrequency signal (244) is a first radiofrequency signal;
the controller-transmitter (220) further comprises a fifth circuit configured to transmit a second radiofrequency signal (242) to the receiver-stimulator (210); and
the receiver-stimulator (210) further comprises an energy-harvesting circuit configured to receive the second radiofrequency signal (242) and convert the second radiofrequency signal to electrical energy.

8. The system of claim 7 wherein the receiver-stimulator (210) further comprises a controller configured to control a timing of the delivery of the electrical energy from the third circuit (212) to the cardiac tissue, and wherein the controller is configured to be at least partially powered by the electrical energy from the energy-harvesting circuit.

9. The system of claim 7 wherein the receiver-stimulator (210) further comprises:
a physiological sensor (218) configured to sense a physiological parameter of the patient, and wherein the physiological sensor (218) is configured to be at least partially powered by the electrical energy from the energy-harvesting circuit; and
a controller configured to (a) receive the electrical energy from the energy-harvesting circuit and the physiological parameter from the physiological sensor and (b) control the fourth circuit (217) to produce the first radiofrequency signal (244) including encoded information about the physiological parameter.

10. The system of claim 1, wherein the receiver-stimulator (210) further comprises:
a storage unit (772,774) electrically coupled to the third circuit (770) and configured to store at least a portion of the electrical energy;
a switch (780) configured to selectively electrically couple the storage unit to the electrode; and
a controller (716) electrically coupled to the switch, wherein the controller is configured to control the switch to deliver the electrical energy from the storage unit to the electrode for output to the tissue as a first electrical pulse (894) and a second electrical pulse (896),
wherein the second electrical pulse is configured to stimulate the tissue, and
wherein the controller-transmitter is configured to detect the first electrical pulse and to cease transmission of the acoustic signal in response to detecting the first electrical pulse.

11. The system of claim 10 wherein the first electrical pulse does not stimulate the tissue.

12. The system of claim 10 wherein the first electrical pulse has a shorter pulse width than the second electrical pulse.

13. The system of claim 10, wherein the controller-transmitter (220) is configured to encode a cardiac interval for the cardiac tissue in the acoustic signal (240), and wherein the controller of the receiver-stimulator (210) is further configured to-
sense a depolarization of the cardiac tissue;
detect the cardiac interval in the acoustic signal;
initiate a timer for the cardiac interval; and
if the depolarization is not sensed during the cardiac interval, control the switch to deliver the second stimulation pulse after the timer has elapsed.

14. The system of claim 10 wherein the controller is configured to control the switch to deliver the first stimulation pulse based on a charge state of the storage unit.

15. The system of claim 10 wherein the controller-transmitter is configured to detect the second electrical pulse and to again transmit the acoustic signal to the receiver-stimulator in response to detecting the second electrical pulse.

## Patentansprüche

1. System (200) zum Stimulieren von Herzgewebe eines Patienten, umfassend:
einen Controller-Sender (220), der eine erste Schaltung (227) und eine zweite Schaltung (229) enthält, wobei die erste Schaltung dazu konfiguriert ist, ein akustisches Signal (240) zu senden, und wobei die zweite Schaltung (229) dazu konfiguriert ist, ein Hochfrequenzsignal (244) zu empfangen; und
einen Empfänger-Stimulator (210), der dazu konfiguriert ist, in den Patienten in der Nähe des Herzgewebes implantiert zu werden, wobei der Empfänger- Stimulator (210) Folgendes enthält:
eine Elektrode (213);
eine dritte Schaltung (212), die funktionsmäßig mit der Elektrode gekoppelt und dazu konfiguriert ist, mindestens einen Teil des akustischen Signals (240) zu empfangen und das akustische Signal (240) in elektrische Energie zur Abgabe an das Herzgewebe über die Elektrode (213) umzuwandeln, um das Herzgewebe elektrisch zu stimulieren; und
eine vierte Schaltung (217), die dazu konfiguriert ist, das Hochfrequenzsignal (244) an die zweite Schaltung (229) des Controller-Senders (220) zu senden;
wobei der Controller-Sender (220) dazu konfiguriert ist, das akustische Signal basierend auf dem Hochfrequenzsignal (244) zu modifizieren, um die elektrische Energie, die über die Elektrode (213) an das Herzgewebe abgegeben wird, zu steuern, um die elektrische Stimulation des Herzgewebes zu steuern.

2. System nach Anspruch 1, wobei der Empfänger- Stimulator (210) ferner einen physiologischen Sensor (218) umfasst, der dazu konfiguriert ist, einen physiologischen Parameter des Patienten zu erfassen, und wobei das Hochfrequenzsignal (244) Informationen über den erfassten physiologischen Parameter enthält.

3. System nach Anspruch 2, wobei der erfasste physiologische Parameter ein intrakardiales Elektrogramm (IEGM) ist oder wobei der erfasste physiologische Parameter ein Herzgeräusch ist.

4. System nach Anspruch 2, wobei der Controller-Sender ferner einen Controller umfasst, der elektrisch mit der ersten Schaltung (227) und der zweiten Schaltung (229) gekoppelt ist, wobei der Controller dazu konfiguriert ist, die erste Schaltung zu steuern, um das akustische Signal mindestens basierend auf dem erfassten physiologischen Parameter zu senden.

5. System nach Anspruch 1, wobei das Hochfrequenzsignal (244) Informationen über einen Zustand des Empfänger-Stimulators (210) enthält.

6. System nach Anspruch 1, wobei das Hochfrequenzsignal (244) Informationen über eine Effizienz der Abgabe der elektrischen Energie an das Herzgewebe enthält.

7. System nach Anspruch 1, wobei
das Hochfrequenzsignal (244) ein erstes Hochfrequenzsignal ist;
der Controller-Sender (220) ferner eine fünfte Schaltung umfasst, die dazu konfiguriert ist, ein zweites Hochfrequenzsignal (242) an den Empfänger-Stimulator (210) zu senden; und
der Empfänger-Stimulator (210) ferner eine Energiegewinnungsschaltung umfasst, die dazu konfiguriert ist, das zweite Hochfrequenzsignal (242) zu empfangen und das zweite Hochfrequenzsignal in elektrische Energie umzuwandeln.

8. System nach Anspruch 7, wobei der Empfänger-Stimulator (210) ferner einen Controller umfasst, der dazu konfiguriert ist, den Zeitpunkt der Abgabe der elektrischen Energie von der dritten Schaltung (212) an das Herzgewebe zu steuern, und wobei der Controller dazu konfiguriert ist, mindestens teilweise durch die elektrische Energie aus der Energiegewinnungsschaltung mit Strom versorgt zu werden.

9. System nach Anspruch 7, wobei der Empfänger-Stimulator (210) ferner Folgendes umfasst:
einen physiologischen Sensor (218), der dazu konfiguriert ist, einen physiologischen Parameter des Patienten zu erfassen, und wobei der physiologische Sensor (218) dazu konfiguriert ist, mindestens teilweise durch die elektrische Energie aus der Energiegewinnungsschaltung mit Strom versorgt zu werden; und
einen Controller, der dazu konfiguriert ist, dass er (a) die elektrische Energie von der Energiegewinnungsschaltung und den physiologischen Parameter vom physiologischen Sensor empfängt und (b) die vierte Schaltung (217) steuert, um das erste Hochfrequenzsignal (244) zu erzeugen, das codierte Informationen über den physiologischen Parameter enthält.

10. System nach Anspruch 1, wobei der Empfänger-Stimulator (210) ferner Folgendes umfasst:
eine Speichereinheit (772, 774), die elektrisch mit der dritten Schaltung (770) gekoppelt und dazu konfiguriert ist, mindestens einen Teil der elektrischen Energie zu speichern;
einen Schalter (780), der dazu konfiguriert ist, die Speichereinheit auswählbar elektrisch mit der Elektrode zu koppeln; und
einen Controller (716), der elektrisch mit dem Schalter gekoppelt ist, wobei der Controller dazu konfiguriert ist, den Schalter zu steuern, um die elektrische Energie von der Speichereinheit an die Elektrode zur Ausgabe an das Gewebe als ersten elektrischen Impuls (894) und zweiten elektrischen Impuls (896) abzugeben,
wobei der zweite elektrische Impuls dazu konfiguriert ist, das Gewebe zu stimulieren, und
wobei der Controller-Sender dazu konfiguriert ist, den ersten elektrischen Impuls zu erkennen und das Senden des akustischen Signals als Reaktion auf das Erkennen des ersten elektrischen Impulses zu unterbrechen.

11. System nach Anspruch 10, wobei der erste elektrische Impuls das Gewebe nicht stimuliert.

12. System nach Anspruch 10, wobei der erste elektrische Impuls eine kürzere Impulsbreite aufweist als der zweite elektrische Impuls.

13. System nach Anspruch 10, wobei der Controller-Sender (220) dazu konfiguriert ist, ein Herzintervall für das Herzgewebe in das akustische Signal (240) zu codieren, und wobei der Controller des Empfänger-Stimulators (210) ferner zu Folgendem konfiguriert ist:
Erfassen einer Depolarisation des Herzgewebes;
Erkennen des Herzintervalls im akustischen Signal;
Initiieren eines Zeitgebers für das Herzintervall; und
falls während des Herzintervalls keine Depolarisation erfasst wird, Ansteuern des Schalters, um den zweiten Stimulationsimpuls abzugeben, nachdem der Zeitgeber abgelaufen ist.

14. System nach Anspruch 10, wobei der Controller dazu konfiguriert ist, den Schalter zu steuern, um den ersten Stimulationsimpuls basierend auf einem Ladezustand der Speichereinheit abzugeben.

15. System nach Anspruch 10, wobei der Controller-Sender dazu konfiguriert ist, den zweiten elektrischen Impuls zu erkennen und als Reaktion auf das Erkennen des zweiten elektrischen Impulses das akustische Signal erneut an den Empfänger-Stimulator zu senden.

## Revendications

1. Système (200) destiné à stimuler un tissu cardiaque d'un patient, comprenant :
un dispositif de commande-émetteur (220) incluant un premier circuit (227) et un deuxième circuit (229), dans lequel le premier circuit est configuré pour émettre un signal acoustique (240), et dans lequel le deuxième circuit (229) est configuré pour recevoir un signal radiofréquence (244) ; et
un récepteur-stimulateur (210) configuré pour être implanté dans le patient à proximité du tissu cardiaque, dans lequel le récepteur-stimulateur (210) inclut -
une électrode (213) ;
un troisième circuit (212) couplé de manière fonctionnelle à l'électrode et configuré pour recevoir au moins une partie du signal acoustique (240) et convertir le signal acoustique (240) en énergie électrique destinée à être délivrée au tissu cardiaque via l'électrode (213) pour stimuler électriquement le tissu cardiaque ; et
un quatrième circuit (217) configuré pour émettre le signal radiofréquence (244) au deuxième circuit (229) du dispositif de commande-émetteur (220) ;
dans lequel le dispositif de commande-émetteur (220) est configuré pour modifier le signal acoustique sur la base du signal radiofréquence (244) pour commander l'énergie électrique délivrée au tissu cardiaque via l'électrode (213) pour commander la stimulation électrique du tissu cardiaque.

2. Système selon la revendication 1 dans lequel le récepteur-stimulateur (210) comprend en outre un capteur physiologique (218) configuré pour capter un paramètre physiologique du patient, et dans lequel le signal radiofréquence (244) inclut des informations sur le paramètre physiologique capté.

3. Système selon la revendication 2 dans lequel le paramètre physiologique capté est un électrocardiogramme intracardiaque (ECGI) ou dans lequel le paramètre physiologique capté est un son cardiaque.

4. Système selon la revendication 2 dans lequel le dispositif de commande-émetteur comprend en outre un dispositif de commande couplé électriquement au premier circuit (227) et au deuxième circuit (229), dans lequel le dispositif de commande est configuré pour commander le premier circuit pour émettre le signal acoustique sur la base au moins en partie du paramètre physiologique capté.

5. Système selon la revendication 1 dans lequel le signal radiofréquence (244) inclut des informations sur un statut du récepteur-stimulateur (210).

6. Système selon la revendication 1 dans lequel le signal radiofréquence (244) inclut des informations sur une efficacité de délivrance de l'énergie électrique au tissu cardiaque.

7. Système selon la revendication 1 dans lequel
le signal radiofréquence (244) est un premier signal radiofréquence ;
le dispositif de commande-émetteur (220) comprend en outre un cinquième circuit configuré pour émettre un deuxième signal radiofréquence (242) au récepteur-stimulateur (210) ; et
le récepteur-stimulateur (210) comprend en outre un circuit de collecte d'énergie configuré pour recevoir le deuxième signal radiofréquence (242) et convertir le deuxième signal radiofréquence en énergie électrique.

8. Système selon la revendication 7 dans lequel le récepteur-stimulateur (210) comprend en outre un dispositif de commande pour commander une synchronisation de la délivrance de l'énergie électrique du troisième circuit (212) au tissu cardiaque, et dans lequel le dispositif de commande est configuré pour être au moins partiellement alimenté en puissance par l'énergie électrique du circuit de collecte d'énergie.

9. Système selon la revendication 7 dans lequel le récepteur-stimulateur (210) comprend en outre :
un capteur physiologique (218) configuré pour capter un paramètre physiologique du patient, et dans lequel le capteur physiologique (218) est configuré pour être au moins partiellement alimenté en puissance par l'énergie électrique du circuit de collecte d'énergie ; et
un dispositif de commande pour (a) recevoir l'énergie électrique du circuit de collecte d'énergie et le paramètre physiologique du capteur physiologique et (b) commander le quatrième circuit (217) pour produire le premier signal radiofréquence (244) incluant des informations codées sur le paramètre physiologique.

10. Système selon la revendication 1, dans lequel le récepteur-stimulateur (210) comprend en outre :
une unité de stockage (772, 774) couplée électriquement au troisième circuit (770) et configurée pour stocker au moins une partie de l'énergie électrique ;
un commutateur (780) configuré pour coupler sélectivement électriquement l'unité de stockage à l'électrode ; et
un dispositif de commande (716) couplé électriquement au commutateur, dans lequel le dispositif de commande est configuré pour commander le commutateur pour délivrer l'énergie électrique de l'unité de stockage à l'électrode pour une sortie vers le tissu comme une première impulsion électrique (894) et une deuxième impulsion électrique (896),
dans lequel la deuxième impulsion électrique est configurée pour stimuler le tissu, et
dans lequel le dispositif de commande-émetteur est configuré pour détecter la première impulsion électrique et pour cesser l'émission du signal acoustique en réponse à la détection de la première impulsion électrique.

11. Système selon la revendication 10 dans lequel la première impulsion électrique ne stimule pas le tissu.

12. Système selon la revendication 10 dans lequel la première impulsion électrique a une largeur d'impulsion plus courte que la deuxième impulsion électrique.

13. Système selon la revendication 10, dans lequel le dispositif de commande-émetteur (220) est configuré pour coder un intervalle cardiaque pour le tissu cardiaque dans le signal acoustique (240), et dans lequel le dispositif de commande du récepteur-stimulateur (210) est configuré en outre pour -
capter une dépolarisation du tissu cardiaque ; détecter l'intervalle cardiaque dans le signal acoustique ;
déclencher un minuteur pour l'intervalle cardiaque ; et
si la dépolarisation n'est pas captée pendant l'intervalle cardiaque, commander le commutateur pour qu'il délivre la deuxième impulsion de stimulation après que le minuteur s'est écoulé.

14. Système selon la revendication 10 dans lequel le dispositif de commande est configuré pour commander le commutateur pour délivrer la première impulsion de stimulation sur la base d'un état de charge de l'unité de stockage.

15. Système selon la revendication 10 dans lequel le dispositif de commande-émetteur est configuré pour détecter la deuxième impulsion électrique et pour émettre à nouveau le signal acoustique vers le récepteur-stimulateur en réponse à la détection de la deuxième impulsion électrique.
